# EUROPEAN PATENT APPLICATION

(11) **EP 1 992 318 A1**
(43) Date of publication of application: **19.11.2008**
(21) Application number: 08156337.1
(22) Date of filing: 16.05.2008
(51) Int. Cl.: A61F 13/20, A61F 13/84

(54) **Nasal tampon**

(30) Priority: 16.05.2007 IT FI20070115
(71) Applicant: Notarbartolo Soluzioni Ospedaliere S.r.l., 50133 Firenze (IT)
(72) Inventor: Turelli, Massimo, 50132, FIRENZE (IT)
(74) Representative: Gervasi, Gemma

(57) **Abstract**

A nasal tampon is described comprising a sheath made of waterproof material provided with through holes on its surface, which facilitates extraction by sliding.

## Description

### Field of the invention

The present invention refers to the field of medical equipment.

### State of the art

As is known, often in nose operations but also in traumatology and control of epistaxis of various types, it is necessary to perform anterior nasal packing in order to arrest the haemorrhage.

Nasal tampons of various shapes and sizes suitable for the above-mentioned purpose are widely known; these are made of material which expands when wetted and are able to perform haemostasis by compression.

The tampons normally used are generally effective in guaranteeing haemostasis but have the drawback of adhering in a more or less tenacious manner to the wounds or to the nasal mucosa. The difficult and painful extraction of the tampons facilitates the onset of epistaxis and makes it difficult for the patient to relax, a necessary condition for complication-free tampon removal.

### Brief description of the figure

The figure shown schematically illustrates a nasal tampon according to the invention.

### Summary of the invention

Nasal tampon comprising a sheath made of waterproof material provided with holes on its surface.

### Detailed disclosure of the invention

The present invention allows the above-mentioned drawbacks to be overcome by producing a nasal tampon that can be easily extracted from the nasal passages without pain and resistance. In practical terms this means: complete pain-free tampon tolerance, a significant reduction in packing and observation time with haemostatic effectiveness identical to that of the tampons currently available on the market.

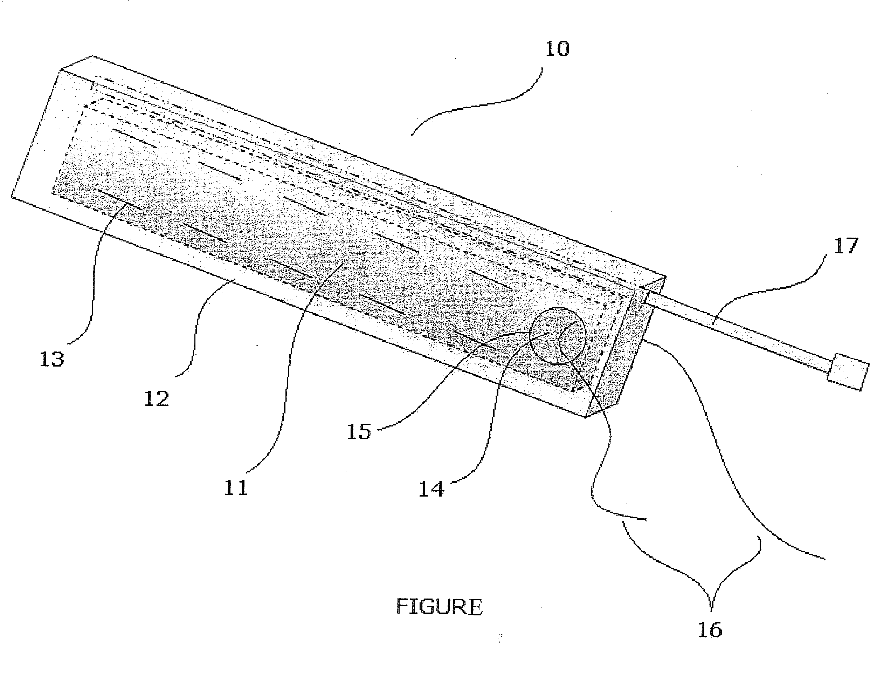

As can be seen from figure 1, a nasal tampon 10 according to the invention comprises: a body 11 made of material with absorbing capacity, a sheath 12 which envelops the above-mentioned body 11 and which is provided with through holes 13 along its surface.

Said body 11 is for example a nasal splint for anterior nasal packing or other expansion device for nasal haemostasis and is made of the normal materials used for traditional nasal tampons such as medical polyurethane or other analogous products.

The sheath 12 consists of a waterproof material which does not adhere to the nasal mucosa and the raw nasal areas during the healing process and therefore slides out in an absolutely trauma-free manner when removed by simply pulling.

Said sheath can for example consist of medical grade polyurethane, latex, latex-free material, silicone or other waterproof material for medical use.

The thickness of the sheath 12 is more or less equivalent to that of surgical gloves, normally between 20 - 80 microns, preferably 40 microns.

The sheath is shaped so as to adapt perfectly to the body retained in it once expanded and will have rounded edges to avoid traumatising the nasal mucosa.

The sheath will be essentially cylindrical in shape (like the finger of a hand) with the two bases closed if necessary by means of any suitable sealing process provided that it is atraumatic.

The holes 13, in any number, can have various shapes and sizes and can be arranged as deemed most appropriate, preferably along the main axis of the device.

If preferred, at one end the sheath can have a portion reinforced with respect to the rest of the sheath and provided with a through hole 14 via which a thread 16 passes in order to facilitate recovery of the tampon; said thread is also passed through the absorbing material contained in the sheath which will obviously also be perforated.

Said thread will preferably be a non-absorbable braided suture thread, for example n ° 0 or 1 with length as required (for example 20 centimetres).

If preferred, the nasal tampon according to the invention can also comprise a tube 17 of varying length which may or may not be provided with fissures. Said tube, which can be pull-out, will be contained inside the sheath and arranged preferably along the main axis of the absorbing body 11 contained in it. The tube will be provided with Luer Lock or analogous connector to allow the absorbing body to be wetted with saline solution or medicaments.

The tube is preferably made of any plastic material such as PVC, silicone, latex, polyurethane and similar on condition that it is medical grade; medical grade PVC is preferred.

Production of the tampon according to the invention is extremely simple.

The absorbing material 11, compressed, and the optional tube provided with Luer Lock or analogous connector, is inserted inside the sheath 12 until the latter covers it completely without gaps or apertures with the exception of the holes 13 as deescribed. Lastly, if required, the tampon recovery thread is applied, passing it through the hole of the sheath provided for the purpose and through the tampon.

Use of the nasal tampon according to the invention is analogously evident.

The tampon inserted in the nasal passages performs its haemostatic action by compression, once expanded. The holes 13 in the sheath guarantee, by drainage, the right expansion and therefore packing capacity. After maintaining the tampon in situ for the necessary time, the device can be removed by simply pulling the recovery thread 16; due to the sheath component material, the tampon will slide easily and painlessly through the nasal passage without resistance.

The nasal tampon according to the invention can be produced in different formats like the tampons currently available on the market, and in the form of kits complete with syringe pre-filled with saline solution for use in accident and emergency departments and if necessary equipped with all the accessories necessary for prompt intervention.

## Claims

1. Nasal tampon comprising a sheath made of waterproof material provided with through holes on its surface.

2. Nasal tampon (10) as claimed in claim 1 comprising: a body (11) made of material with absorbing capacity, a sheath (12) which envelops said above-mentioned body (11) and which is provided with through holes (13) along its surface.

3. Nasal tampon as claimed in claim 2 wherein said body (11) is a nasal splint for anterior nasal packing or other expansion device for nasal haemostasis consisting of the normal materials used for traditional nasal tampons.

4. Nasal tampon as claimed in claim 3 wherein said materials are medical polyurethane or other analogous products.

5. Nasal tampon as claimed in claims 1 - 4 wherein said sheath (12) consists of a waterproof material which does not adhere to the nasal mucosa and to the raw nasal areas during the healing process.

6. Nasal tampon as claimed in claim 5 wherein said sheath consists of polyurethane, latex, latex-free material, silicone or other waterproof material for medical use.

7. Tampon as claimed in claim 6 wherein said sheath has a thickness of approximately 20 - 80 microns.

8. Nasal tampon as claimed in claims 1 - 7 wherein the holes (13), in any number and of varying shape and size, are arranged along the main axis of the device.

9. Nasal tampon as claimed in the preceding claims wherein one end of the sheath has a reinforcing edge (15) with through hole (14) via which a tampon recovery thread (16) passes which also passes through the body (11) contained in the sheath (12).

10. Tampon as claimed in claim 9 wherein said thread is a non-absorbable braided suture thread of any length.

11. Nasal tampon as claimed in claims 1-10 wherein inside the sheath (12) and along the main axis of the absorbing body contained in it, a removable tube (17) is inserted of varying length with or without fissures provided with Luer Lock or analogous connector.

12. Kit comprising one or more nasal tampons as claimed in claims 1-11, with identical or different dimensions, and a syringe pre-filled with saline solution and optionally equipped with all the accessories necessary for prompt intervention.

13. Use of a nasal tampon as claimed in claims 1 - 11 in nose operations and in traumatology and control of epistaxis of various types.
